# EUROPEAN PATENT APPLICATION

(11) **EP 3 470 091 A1**
(43) Date of publication of application: **17.04.2019**
(21) Application number: 17810420.4
(22) Date of filing: 09.06.2017
(51) Int. Cl.: A61K 51/04, A61K 49/00, A61K 49/04

(54) **NON-INVASIVE DIAGNOSTIC IMAGING AGENT FOR HEART DISEASE**

(30) Priority: 10.06.2016 JP 2016115806
(71) Applicant: Nihon Medi-Physics Co., Ltd., Tokyo 136-0075 (JP)
(72) Inventor: MAYA, Yoshifumi, Tokyo 136-0075 (JP)
(74) Representative: TBK
(86) International application number: PCT/JP2017/021442
(87) International publication number: WO 2017/213247

(57) **Abstract**

The present invention addresses the problem of providing a technique for detecting a lesion of a heart disease in a non-invasive manner. The present invention provides a non-invasive diagnostic imaging agent for a heart disease, which contains a radioactively labeled compound capable of binding to an aldosterone synthase or a salt thereof as an active ingredient.

## Description

### Technical Field

The present invention relates to a non-invasive diagnostic imaging agent for a heart disease.

### Background Art

In chronic heart failure, left ventricular remodeling such as ventricular hypertrophy and fibrosis progresses in the heart. It has been reported that the activation of the renin-angiotensin-aldosterone system (RAAS) is associated with this process. RAAS is a regulatory mechanism of the circulatory system that regulates blood pressure in the body. In addition to this RAAS of the systemic circulatory system, data has recently been reported, indicating that the RAAS or aldosterone synthesis system is present at the heart locally.

For example, non-Patent Literature 1 states that blood collected by catheterization of the heart showed that aldosterone was synthesized or secreted in the human failing heart.

Non-Patent Literature 2 discloses that a study using an autopsied heart revealed an increased gene expression of an aldosterone synthase CYP11B2. This literature further discloses that the increased expression of the CYP11B2 gene is related to myocardial fibrosis or cardiac dysfunction.

Attempts have been made to develop various compounds that selectively inhibit CYP11B2 such that cardiovascular diseases are treated by selectively inhibiting CYP11B2 (Patent Literatures 1 to 3 and non-Patent Literatures 3 to 5).

Although not intended for cardiovascular diseases, various radioactive compounds that exhibit high selectivity for CYP11B2 have also been developed for the purpose of visualizing a lesion in the adrenal gland and enabling diagnostic imaging of primary aldosteronism (Patent Literatures 4 to 11 and non-Patent Literature 6).

### Citation List

### Patent Literature

Patent Literature 1: Japanese Patent Laid-Open (Kohyo) No. 2013-512271
Patent Literature 2: Japanese Patent Laid-Open (Kohyo) No. 2011-520799
Patent Literature 3: Japanese Patent Laid-Open (Kohyo) No. 2014-526539
Patent Literature 4: Japanese Patent Laid-Open (Kohyo) No. 2013-534911
Patent Literature 5: Japanese Patent Laid-Open (Kokai) No. 2014-129315
Patent Literature 6: Japanese Patent Laid-Open (Kokai) No. 2015-093831
Patent Literature 7: Japanese Patent Laid-Open (Kokai) No. 2015-093832
Patent Literature 8: Japanese Patent Laid-Open (Kokai) No. 2015-093833
Patent Literature 9: Japanese Patent Laid-Open (Kokai) No. 2015-110563
Patent Literature 10: Japanese Patent Laid-Open (Kokai) No. 2015-193545
Patent Literature 11: International Publication No. WO 2015/199205

### Non-Patent Literature

Non-Patent Literature 1: Mizuno, Y. et al., Circulation, vol. 103, p. 72-7 (2001)
Non-Patent Literature 2: Satoh, M. et al., Clinical Science, vol. 102, p. 381-386 (2002)
Non-Patent Literature 3: Grombein, C. M., European Journal of Medicinal Chemistry, vol. 89, p. 597-605 (2015)
Non-Patent Literature 4: Grombein, C. M., European Journal of Medicinal Chemistry, vol. 90, p. 788-796 (2015)
Non-Patent Literature 5: Martin, R. E. et al., Journal of Medicinal Chemistry, vol. 58, p. 8054-8065 (2015)
Non-Patent Literature 6: Abe, T., Journal of Clinical Endocrinol Metabolism, vol. 101, p. 1008-1015 (2016)

### Summary of Invention

There might be a possibility that the detection of expression of CYP11B2 in the heart leads to the evaluation of course of progression of heart diseases such as heart failure. However, no non-invasive method exists that can detect such lesions at preset.

One example of the method for detecting a change at molecular level *in vivo* is nuclear medicine diagnosis using single-photon emission computed tomography (SPECT) and positron emission tomography (PET). Patent Literatures 4 to 11 and non-Patent Literature 6 have reported tracers for SPECT or PET that exhibit high selectivity for CYP11B2. However, these tracers target the adrenal gland and have not yet been found to be suitable for imaging in the heart.

No finding has been obtained as to whether or not the local expression of CYP11B2 in the heart of heart disease patients is increased to a level that permits visualization by nuclear medicine diagnosis.

The present invention has been made in light of the circumstances described above and is directed to providing a technique for detecting a lesion of a heart disease in a non-invasive manner.

The present invention provides a non-invasive diagnostic imaging agent for a heart disease, comprising a radioactively labeled compound capable of binding to an aldosterone synthase or a salt thereof as an active ingredient.

According to the present invention, a lesion of a heart disease can be detected in a non-invasive manner.

### Brief Description of Drawings

The object mentioned above and other objects, features and advantages of the present invention will become more apparent upon reading the following preferred embodiments and accompanying drawings.

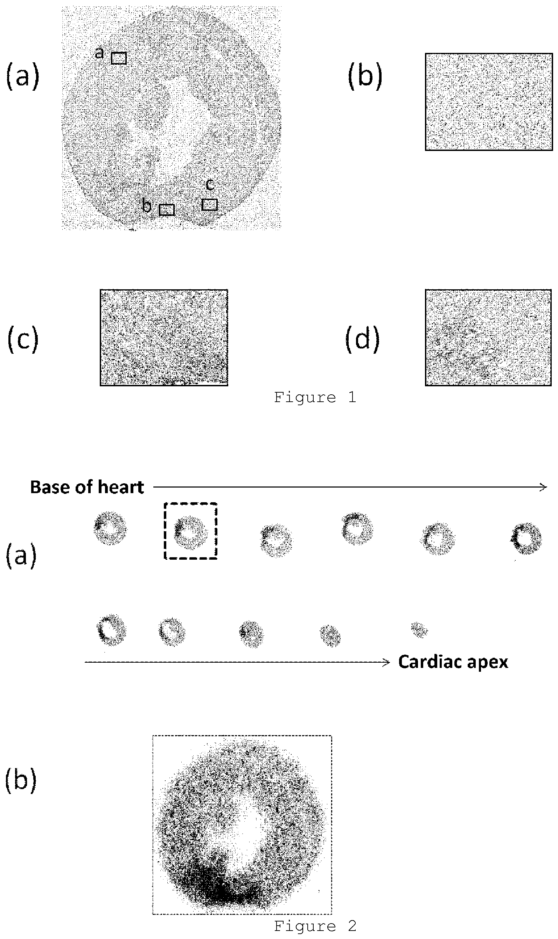

Figure 1 represents views showing a result of immunostaining of CYP11B2 using a heart tissue section of an ischemic heart disease model rat. Figure 1(a) is an overall view of the section, Figure 1(b) is an enlarged view of a non-ischemic site, and each of Figures 1(c) and 1(d) is an enlarged view of an ischemia reperfusion site.
Figure 2 represents views showing a result of autoradiogram of an individual given 6-chloro-5-fluoro-1-(2-[¹⁸F]fluoroethyl)-2-[5-(imidazol-1-ylmethyl)pyridin-3-yl]benzimidazole (Compound [¹⁸F] 1). Figure 2(a) represents views showing a series of autoradiograms of sections arranged from the base of the heart toward the cardiac apex, and Figure 2(b) is an enlarged view after rotation by 90 degrees of the autoradiogram surrounded with the broken line in Figure 2(a).

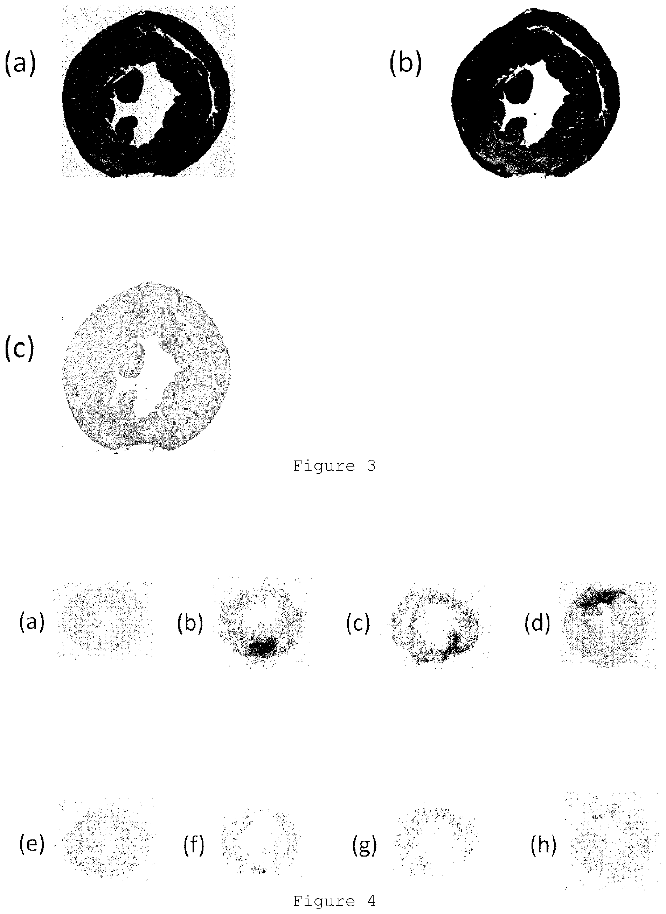

Figure 3 represents views showing results of staining a section adjacent to the heart tissue section shown in Figure 2(b). Figure 3(a) is a view showing a result of HE staining, Figure 3(b) is a view showing a result of Masson trichrome staining, and Figure 3(c) is a view showing a result of immunostaining of CYP11B2.
Figure 4 represents views showing results of *in vitro* autoradiography using 6-chloro-5-fluoro-1-(2-[¹⁸F]fluoroethyl)-2-[5-(imidazol-1-ylmethyl)pyridin-3-yl]benzimidazole (Compound [¹⁸F] 1). Figure 4(a) is a view showing a result of immersing a heart tissue section of a normal rat in a solution containing 6-chloro-5-fluoro-1-(2-[¹⁸F]fluoroethyl)-2-[5-(imidazol-1-ylmethyl)pyridin-3-yl]benzimidazole (Compound [¹⁸F] 1). Each of Figures 4(b) to 4(d) is a view showing a result of immersing a heart tissue section of an ischemic heart disease model rat in a solution containing 6-chloro-5-fluoro-1-(2-[¹⁸F]fluoroethyl)-2-[5-(imidazol-1-ylmethyl)pyridin-3-yl]benzimidazole (Compound [¹⁸F] 1). Figure 4(e) is a view showing a result of immersing a heart tissue section of the normal rat in a solution containing 6-chloro-5-fluoro-1-(2-[¹⁸F]fluoroethyl)-2-[5-(imidazol-1-ylmethyl)pyridin-3-yl]benzimidazole (Compound [¹⁸F] 1) and 5 µmol/L 6-chloro-5-fluoro-1-(2-fluoroethyl)-2-[5-(imidazol-1-ylmethyl)pyridin-3-yl]benzimidazole (Compound 1). Each of Figures 4(f) to 4(h) is a view showing a result of immersing a heart tissue section of the ischemic heart disease model rat in a solution containing 6-chloro-5-fluoro-1-(2-[¹⁸F]fluoroethyl)-2-[5-(imidazol-1-ylmethyl)pyridin-3-yl]benzimidazole (Compound [¹⁸F] 1) and 5 µmol/L 6-chloro-5-fluoro-1-(2-fluoroethyl)-2-[5-(imidazol-1-ylmethyl)pyridin-3-yl]benzimidazole (Compound 1).

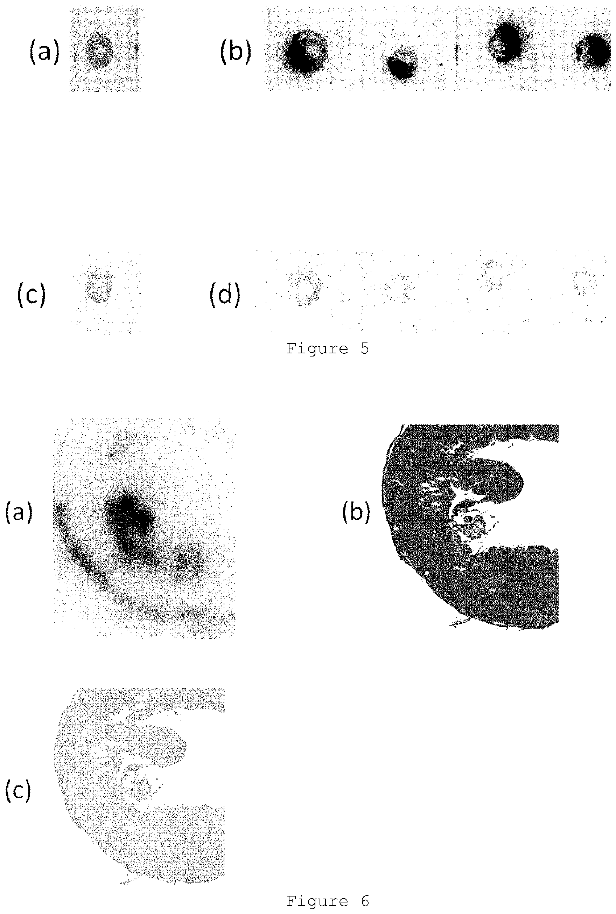

Figure 5 represents views showing results of *in vitro* autoradiography using 1-(2-fluoroethyl)-2-[5-{(imidazol-1-yl)methyl}pyridin-3-yl]-6-[¹²³I] iodobenzimidazole (Compound [¹²³I] 2). Figure 5(a) is a view showing a result of immersing a heart tissue section of a normal rat in a solution containing 1-(2-fluoroethyl)-2-[5-{(imidazol-1-yl)methyl}pyridin-3-yl]-6-[¹²³I] iodobenzimidazole (Compound [¹²³I] 2). Figure 5(b) represents views showing results of immersing heart tissue sections of an ischemic heart disease model rat in a solution containing 1-(2-fluoroethyl)-2-[5-{(imidazol-1-yl)methyl}pyridin-3-yl]-6-[¹²³I]iodobenzimidazole (Compound [¹²³I] 2). Figure 5(c) is a view showing a result of immersing a heart tissue section of the normal rat in a solution containing 1-(2-fluoroethyl)-2-[5-{(imidazol-1-yl)methyl}pyridin-3-yl]-6-[¹²³I] iodobenzimidazole (Compound [¹²³I] 2) and 5 (µmol/L 1-(2-fluoroethyl)-2-[5-{(imidazol-1-yl)methyl}pyridin-3-yl]-6-iodobenzimidazole (Compound 2). Figure 5(d) represents views showing results of immersing heart tissue sections of the ischemic heart disease model rat in a solution containing 1-(2-fluoroethyl)-2-[5-{(imidazol-l-yl)methyl}pyridin-3-yl]-6-[¹²³I] iodobenzimidazole (Compound [¹²³I] 2) and 5 µmol/L 1-(2-fluoroethyl)-2-[5-{(imidazol-1-yl)methyl}pyridin-3-yl]-6-iodobenzimidazole (Compound 2).
Figure 6 represents views showing results of autoradiography and staining of a heart tissue section of an ischemic heart disease model rat using 1-(2-fluoroethyl)-2-[5-{(imidazol-l-yl)methyl}pyridin-3-yl]-6-[¹²³I] iodobenzimidazole (Compound [¹²³I] 2). Figure 6(a) is a view showing a result of autoradiography, Figure 6(b) is a view showing a result of Masson trichrome staining, and Figure 6(c) is a view showing a result of immunostaining of CYP11B2.

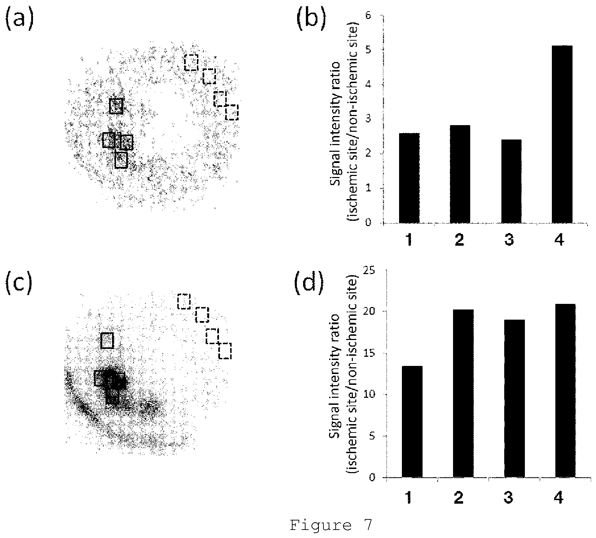

Figure 7 represents views showing comparison of results of autoradiography using 6-chloro-5-fluoro-1-(2-[¹⁸F]fluoroethyl)-2-[5-(imidazol-1-ylmethyl)pyridin-3-yl]benzimidazole (Compound [¹⁸F] 1) and 1-(2-fluoroethyl)-2-[5-{(imidazol-1-yl)methyl}pyridin-3-yl]-6-[¹²³I] iodobenzimidazole (Compound [¹²³I] 2). Figure 7(a) is a view showing an example of region of interest (ROI) established on an autoradiogram of a section immersed in a solution containing 6-chloro-5-fluoro-1-(2-[¹⁸F]fluoroethyl)-2-[5-(imidazol-1-ylmethyl)pyridin-3-yl]benzimidazole (Compound [¹⁸F] 1). Figure 7(b) is a bar graph showing a ratio of ischemia reperfusion site signal intensity/non-ischemic site signal intensity of 6-chloro-5-fluoro-1-(2-[¹⁸F]fluoroethyl)-2-[5-(imidazol-1-ylmethyl)pyridin-3-yl]benzimidazole (Compound [¹⁸F] 1) on an individual basis. Figure 7(c) is a view showing an example of ROI established on an autoradiogram of a section immersed in a solution containing 1-(2-fluoroethyl)-2-[5-{(imidazol-1-yl)methyl}pyridin-3-yl]-6-[¹²³I] iodobenzimidazole (Compound [¹²³I] 2). Figure 7(d) is a bar graph showing a ratio of ischemia reperfusion site signal intensity/non-ischemic site signal intensity of 1-(2-fluoroethyl)-2-[5-{(imidazol-1-yl)methyl}pyridin-3-yl]-6-[¹²³I] iodobenzimidazole (Compound [¹²³I] 2) on an individual basis.

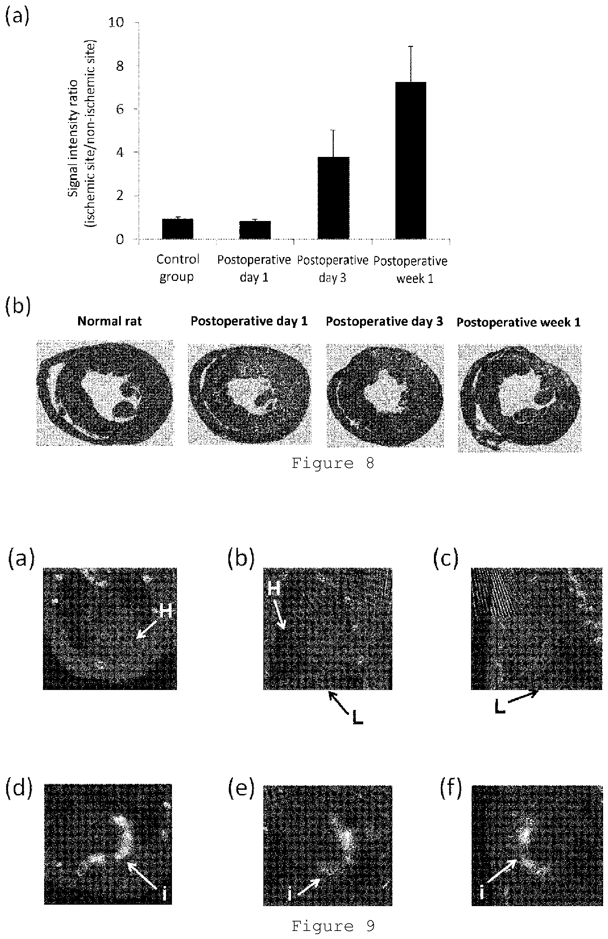

Figure 8 represents views showing results of autoradiography and staining with 1-(2-fluoroethyl)-2-[5-{(imidazol-1-yl)methyl}pyridin-3-yl]-6-[¹²³I] iodobenzimidazole (Compound [¹²³I] 2) using the hearts of a normal rat and rats 1 day, 3 days and 1 week after ischemic heart disease model rat preparation. Figure 8(a) is a bar graph showing a ratio of ischemia reperfusion site signal intensity/non-ischemic site signal intensity, and Figure 8(b) represents views showing results of Masson trichrome staining.
Figure 9 represents views displaying a SPECT image with 1-(2-fluoroethyl)-2-[5-{(imidazol-1-yl)methyl}pyridin-3-yl]-6-[¹²³I]iodobenzimidazole (Compound [¹²³I] 2), which is superimposed with a computed tomography image. Figure 9(a) is a short axis image of a normal rat, Figure 9(b) is a horizontal long axis image of the normal rat, Figure 9(c) is a vertical long axis image of the normal rat, Figure 9(d) is a short axis image of an ischemic heart disease model rat, Figure 9(e) is a horizontal long axis image of the ischemic heart disease model rat, and Figure 9(f) is a vertical long axis image of the ischemic heart disease model rat.

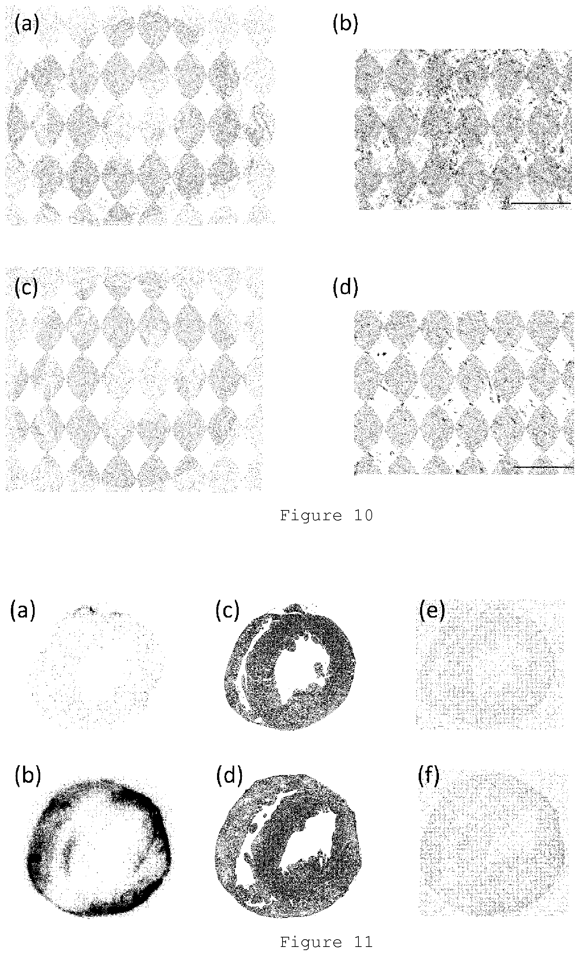

Figure 10 represents views showing results of immunostaining of CYP11B2 using the respective heart tissue sections of a myocarditis model rat and a normal rat. Figure 10(a) is an overall view of the heart tissue section of the myocarditis model rat, Figure 10(b) is an enlarged view of Figure 10(a), Figure 10(c) is an overall view of the heart tissue section of the normal rat, and Figure 10(d) is an enlarged view of Figure 10(c).
Figure 11 represents views showing results of *in vitro* autoradiography with 2-[5-{(1H-imidazol-1-yl)methyl}pyridin-3-yl]-6-[¹²³I]iodo-1-methyl-1H-benz [d] imidazole (Compound [¹²³I] 3). Figure 11(a) is a view showing a result of autoradiography of a heart tissue section of a normal rat, Figure 11(b) is a view showing a result of autoradiography of a heart tissue section of a myocarditis model rat, Figure 11(c) is a view showing a result of Masson trichrome staining of a heart tissue section of the normal rat, Figure 11(d) is a view showing a result of Masson trichrome staining of a heart tissue section of the myocarditis model rat, Figure 11(e) is a view showing a result of immunostaining of CYP11B2 of a heart tissue section of the normal rat, and Figure 11(f) is a view showing a result of immunostaining of CYP11B2 of a heart tissue section of the myocarditis model rat.

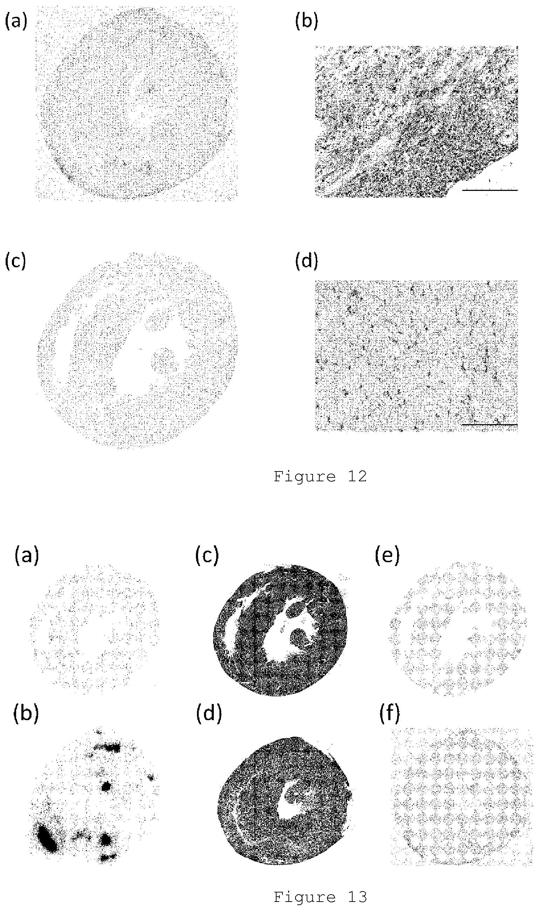

Figure 12 represents views showing results of immunostaining of CYP11B2 using the respective heart tissue sections of a hypertensive heart disease model rat and a normal rat. Figure 12(a) is an overall view of the heart tissue section of the hypertensive heart disease model, Figure 12(b) is an enlarged view of Figure 12(a), Figure 12(c) is an overall view of the heart tissue section of the normal rat, and Figure 12(d) is an enlarged view of Figure 12(c).
Figure 13 represents views showing results of *in vitro* autoradiography with 2-[5-{(1H-imidazol-1-yl)methyl}pyridin-3-yl]-6-[¹²³I]iodo-1-methyl-1H-benz [d] imidazole (Compound [¹²³I] 3). Figure 13(a) is a view showing a result of autoradiography of a heart tissue section of a normal rat, Figure 13(b) is a view showing a result of autoradiography of a heart tissue section of a hypertensive heart disease model rat, Figure 13(c) is a view showing a result of Masson trichrome staining of a heart tissue section of the normal rat, Figure 13(d) is a view showing a result of Masson trichrome staining of a heart tissue section of the hypertensive heart disease model rat, Figure 13(e) is a view showing a result of immunostaining of CYP11B2 of a heart tissue section of the normal rat, and Figure 13(f) is a view showing a result of immunostaining of CYP11B2 of a heart tissue section of the hypertensive heart disease model rat.

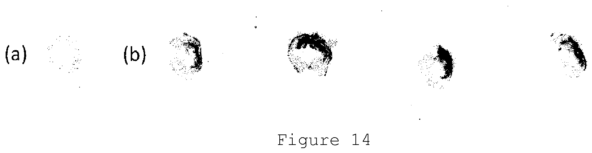

Figure 14 represents views showing results of *in vitro* autoradiography using 2-[5-{(1H-imidazol-1-yl)methyl}pyridin-3-yl]-6-[¹²³I]iodo-1-methyl-1H-benz [d] imidazole (Compound [¹²³I] 3). Figure 14(a) is a view showing a result of immersing a heart tissue section of a normal rat in a solution containing 2-[5-{(1H-imidazol-1-yl)methyl}pyridin-3-yl]-6-[¹²³I]iodo-1-methyl-1H-benz [d] imidazole (Compound [¹²³I] 3). Figure 14(b) represents views showing results of immersing heart tissue sections of an ischemic heart disease model rat in a solution containing 2-[5-{(1H-imidazol-1-yl)methyl}pyridin-3-yl]-6-[¹²³I]iodo-1-methyl-1H-benz[d]imidazole (Compound [¹²³I] 3).

### Description of Embodiments

The present invention provides a non-invasive diagnostic imaging agent for a heart disease, comprising a radioactively labeled compound capable of binding to an aldosterone synthase or a salt thereof as an active ingredient. The diagnostic imaging agent of the present invention can visualize a site having the advanced fibrosis of the heart.

In the present invention, the "non-invasive diagnostic imaging agent" refers to one which is used in nuclear medicine diagnosis and is preferably used in positron emission tomography (PET) or single-photon emission computed tomography (SPECT).

In the present invention, the "heart disease" includes an ischemic heart disease and a non-ischemic heart disease and is preferably a disease caused by the fibrosis of the heart. One example thereof is heart failure.

In the present invention, the "ischemic heart disease" is not limited as long as the ischemic heart disease is a heart disease caused by myocardial ischemia. Examples thereof include coronary heart diseases such as angina pectoris, myocardial infarction, acute coronary syndrome, and ischemic heart failure.

In the present invention, examples of the "non-ischemic heart disease" include myocarditis, hypertensive heart disease, dilated cardiomyopathy, hypertrophic cardiomyopathy, and non-ischemic heart failure.

In the present invention, the "radioactively labeled compound" is not limited as long as the radioactively labeled compound is a compound labeled with a radioisotope for use in nuclear medicine diagnosis. Examples of the radioisotope include carbon-11, fluorine-18, chlorine-34m, bromine-76, iodine-123 and iodine-124. The non-invasive diagnostic imaging agent of the present invention can be used as a diagnostic imaging agent for positron emission tomography in the case of using a positron-emitting radionuclide such as carbon-11, fluorine-18, chlorine-34m or iodine-124 as the radioisotope, and can be used as a diagnostic imaging agent for single-photon emission computed tomography in the case of using iodine-123 as a radioactive halogen atom.

In the present invention, the phrase "capable of binding to an aldosterone synthase" means being capable of binding to human CYP11B2.

In the present invention, the "radioactively labeled compound capable of binding to an aldosterone synthase" is not limited as long as the radioactively labeled compound is capable of binding to human CYP11B2. Examples thereof include radioactively labeled compounds having affinity for adrenocortical adenoma or adrenocortical carcinoma as described in U.S. Patent Application Publication No. 2005/0033060 and Japanese Patent Laid-open (Kohyo) No. 2009-539822, and radioactively labeled compounds having CYP11B2 selectivity as described in Japanese Patent Laid-open (Kohyo) No. 2013-534911, Japanese Patent Laid-Open (Kokai) No. 2014-129315, Japanese Patent Laid-Open (Kokai) No. 2015-093831, Japanese Patent Laid-Open (Kokai) No. 2015-093832, Japanese Patent Laid-Open (Kokai) No. 2015-093833, Japanese Patent Laid-Open (Kokai) No. 2015-110563, Japanese Patent Laid-Open (Kokai) No. 2015-193545, and International Publication No. WO 2015/199205. Alternatively, the radioactively labeled compound capable of binding to an aldosterone synthase may be a compound derived from a compound described in Japanese Patent Laid-open (Kohyo) No. 2013-512271, Japanese Patent Laid-open (Kohyo) No. 2011-520799, Japanese Patent Laid-open (Kohyo) No. 2011-525894, Japanese Patent Laid-open (Kohyo) No. 2012-526774, Japanese Patent Laid-open (Kohyo) No. 2013-510896, Japanese Patent Laid-open (Kohyo) No. 2014-526539, Japanese Patent Laid-open (Kohyo) No. 2014-527077, or Japanese Patent Laid-open (Kohyo) No. 2014-533736 by the replacement of one constituent element with the radioisotope mentioned above.

In the present invention, the radioactively labeled compound is preferably a compound represented by the following general formula (1) described in International Publication No. WO 2015/199205.

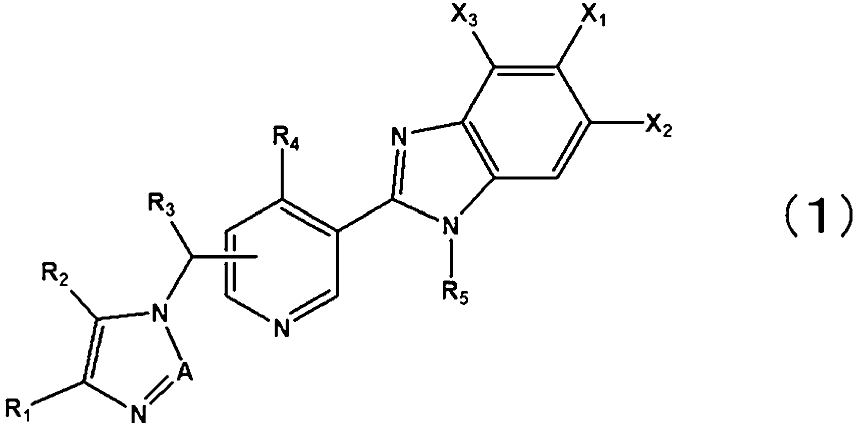

In the general formula (1), R₁ represents a hydrogen atom or CO₂Rₐ. R₂ represents a hydrogen atom, a halogen atom or CO₂Rₐ. R₃ represents a hydrogen atom or a hydroxyalkyl group having 1 to 10 carbon atoms. R₄ represents a hydrogen atom, a hydroxy group or an alkoxy group having 1 to 10 carbon atoms. R₅ represents a chain alkyl group having 1 to 5 carbon atoms in which a hydrogen atom is optionally replaced with a halogen atom, a cyclic alkyl group having 3 to 5 carbon atoms in which a hydrogen atom is optionally replaced with a halogen atom, a hydroxyalkyl group having 1 to 5 carbon atoms, or an o-, p- or m-halobenzyl group. A represents CH or a nitrogen atom. X₁ and X₃ each independently represent a hydrogen atom or a halogen atom. X₂ represents a hydrogen atom, a halogen atom or a nitrile group. At least one of X₁, X₂ and X₃ is a halogen atom.

In the radioactively labeled compound of the general formula (1), the "CO₂Rₐ" is a carboxylic acid ester group. Each Rₐ is independently an alkyl group having 1 to 10 carbon atoms. The alkyl group may be linear or branched and is preferably an alkyl group having 1 to 5 carbon atoms (methyl group, ethyl group, n-propyl group, isopropyl group, n-butyl group, isobutyl group, tert-butyl group, n-pentyl group, isopentyl group, or neopentyl group), more preferably an alkyl group having 1 to 3 carbon atoms (methyl group, ethyl group, n-propyl group, or isopropyl group). The "CO₂Rₐ" is particularly preferably a "carboxylic acid methyl ester group," in which Rₐ is a methyl group.

In the radioactively labeled compound of the general formula (1), the "halogen atom" is a fluorine atom, a chlorine atom, a bromine atom or an iodine atom.

In the compound of the general formula (1), the "hydroxyalkyl group" is a group represented by -(CH₂)ₘOH. In the general formula (1), for example, m for R₃ is an integer of 1 to 10, preferably an integer of 1 to 3. In the general formula (1), m for R₅ is an integer of 1 to 5, preferably an integer of 1 to 3.

In the radioactively labeled compound of the general formula (1), the "alkoxy group" is a group in which a linear or branched alkyl group is bonded to an oxygen atom. Examples thereof preferably include a methoxy group, an ethoxy group, a propoxy group, and an isopropoxy group, among which a methoxy group is more preferred.

In the radioactively labeled compound of the general formula (1), the "chain alkyl group" is a non-cyclic alkyl group and may be linear or branched. Examples thereof preferably include a methyl group, an ethyl group, a propyl group, an isopropyl group, a n-butyl group, an isobutyl group, a sec-butyl group, a tert-butyl group, a n-pentyl group, an isopentyl group, a neopentyl group, and a tert-pentyl group. In these chain alkyl groups, one or two or more hydrogen atoms may be replaced with halogen atom(s) and are preferably replaced with fluorine atom(s). Specific examples thereof include a fluoromethyl group, a 1-fluoroethyl group, a 1,1-difluoroethyl group, a 1,1,1-trifluoroethyl group, and a 1-fluoropropyl group.

In the radioactively labeled compound of the general formula (1), the "cyclic alkyl group" includes a cyclopropyl group, a cyclobutyl group, and a cyclopentyl group. In these cyclic alkyl groups, one or two or more hydrogen atoms may be replaced with halogen atom(s).

In the radioactively labeled compound of the general formula (1), the "halobenzyl group" is a benzyl group in which a hydrogen atom at position 2, 3, or 4 of the benzene ring is replaced with a halogen atom. A benzyl group in which the hydrogen atom at position 2 is replaced with a halogen atom is an o-halobenzyl group. A benzyl group in which the hydrogen atom at position 3 is replaced with a halogen atom is a m-halobenzyl group. A benzyl group in which the hydrogen atom at position 4 is replaced with a halogen atom is a p-halobenzyl group. Among them, a p-halobenzyl group is preferred.

In the radioactively labeled compound of the general formula (1), any one of R₂, R₅, and X₂ contains a radioactive halogen atom. Preferably, the radioactively labeled compound of the general formula (1) has the following configuration (a), (b), (c) or (d):
(a) a radioactive halogen atom is used as a halogen atom in R₂;
(b) R₅ is a group represented by -(CH₂)ₙX₄, and a radioactive halogen atom is used as a halogen atom of X₄;
(c) R₅ is a p-halobenzyl group, and a radioactive halogen atom is used as a halogen atom introduced at position 4 of the benzyl group; and
(d) a radioactive halogen atom is used as a halogen atom of X₂.

In this context, the "radioactive halogen atom" refers to any of fluorine-18, chlorine-34m, bromine-76, iodine-123 and iodine-124.

More preferably, in the general formula (1), R₃ is a hydrogen atom; R₄ is a hydrogen atom or an alkoxy group having 1 to 10 carbon atoms; R₅ is a chain alkyl group having 1 to 5 carbon atoms in which a hydrogen atom is optionally replaced with a halogen atom, a cyclic alkyl group having 3 to 5 carbon atoms, or an o-, p- or m-halobenzyl group; X₂ is a halogen atom; and X₃ is a hydrogen atom. R₅ is more preferably a methyl group, an ethyl group, a propyl group, an isopropyl group, a group represented by -(CH₂)ₙX₄ (wherein n represents an integer of 1 to 5, and X₄ represents a halogen atom), a cyclopropyl group, or a p-halobenzyl group.

Further preferably, in the general formula (1), R₂ is a hydrogen atom or a halogen atom.

Further preferably, in the general formula (1), R₅ is a methyl group, an ethyl group, a group represented by -(CH₂)ₙX₄, or a cyclopropyl group. In the group represented by -(CH₂)ₙX₄, n is preferably an integer of 1 to 3, more preferably 2 or 3, furthermore preferably 2. X₄ is preferably a fluorine atom.

One specific embodiment of the compound according to the present invention is a compound represented by the general formula (2).

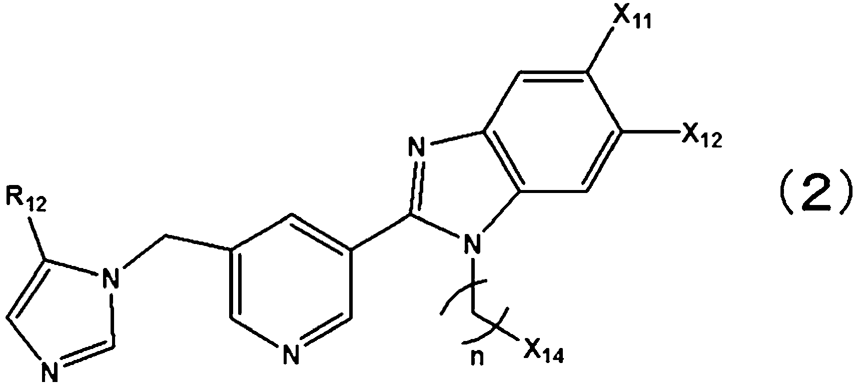

In the general formula (2), R₁₂ represents a hydrogen atom, a halogen atom or CO₂Rₐ. X₁₁ represents a hydrogen atom or a halogen atom. X₁₂ represents a halogen atom. X₁₄ represents a hydrogen atom, a halogen atom or a hydroxy group. n represents an integer of 1 to 5. In the radioactively labeled compound of the general formula (2), the "CO₂Rₐ" is as defined in the general formula (1).

The compound represented by the general formula (2) is a compound of the general formula (1) wherein R₁ is a hydrogen atom; R₂ is a hydrogen atom, a halogen atom, or CO₂Rₐ (wherein Rₐ is an alkyl group having 1 to 10 carbon atoms); each of R₃ and R₄ is a hydrogen atom; R₅ is a chain alkyl group having 1 to 5 carbon atoms, or a group represented by -(CH₂)ₙX₁₄; A is CH; X₁ is a hydrogen atom or a halogen atom; X₂ is a halogen atom; and X₃ is a hydrogen atom. In the general formula (2), R₁₂ may represent a hydrogen atom or CO₂Rₐ and is preferably a hydrogen atom. X₁₄ is preferably a hydrogen atom or a fluorine atom, and n is preferably an integer of 1 to 3.

In the general formula (2), R₁₂, X₁₂, or X₁₄ is a radioactive halogen atom. Preferably, X₁₂ or X₁₄ is a radioactive halogen atom. More preferably, R₁₂ is a hydrogen atom, and X₁₂ or X₁₄ is a radioactive halogen atom. In this case, still more preferably, n is an integer of 1 to 3. The radioactive halogen atom is as defined above.

The compounds represented by the general formulas (1) and (2) can be obtained by methods described in International Publication No. WO 2015/199205.

In the present invention, the "salt" may be one that is pharmaceutically acceptable. The salt can be, for example, a salt derived from an inorganic acid such as hydrochloric acid, hydrobromic acid, sulfuric acid, nitric acid, and phosphoric acid, or an organic acid such as acetic acid, trifluoroacetic acid, maleic acid, succinic acid, mandelic acid, fumaric acid, malonic acid, pyruvic acid, oxalic acid, glycolic acid, salicylic acid, pyranosidyl acids (glucuronic acid, galacturonic acid, etc.), a-hydroxy acids (citric acid, tartaric acid, etc.), amino acids (aspartic acid, glutamic acid, etc.), aromatic acids (benzoic acid, cinnamic acid, etc.), and sulfonic acids (p-toluenesulfonic acid, ethanesulfonic acid, etc.).

The non-invasive diagnostic imaging agent of the present invention is a formulation containing the radioactively labeled compound described above or the salt thereof in a form suitable for administration into a living body. This non-invasive diagnostic imaging agent is preferably administered through a parenteral route, i.e., by injection, and is more preferably an aqueous solution. Such a composition may appropriately contain an additional component such as a pH adjuster, or a pharmaceutically acceptable solubilizer, stabilizer or antioxidant.

### Examples

Hereinafter, the present invention will be described in more detail by way of Examples. However, the present invention is not limited by the contents thereof.

In Examples given below, the names of compounds subjected to experiments were defined as follows.
Compound 1: 6-chloro-5-fluoro-1-(2-fluoroethyl)-2-[5-(imidazol-1-ylmethyl)pyridin-3-yl]benzimidazole Compound [¹⁸F] 1: 6-chloro-5-fluoro-1-(2-[¹⁸F]fluoroethyl)-2-[5-(imidazol-1-ylmethyl)pyridin-3-yl]benzimidazole (a compound represented by the general formula (2) wherein R₁₂ represents a hydrogen atom; X₁₁ represents a fluorine atom; X₁₂ represents a chlorine atom; X₁₄ represents fluorine-18; and n represents an integer of 2)
Compound 2: 1-(2-fluoroethyl)-2-[5-{(imidazol-1-yl)methyl}pyridin-3-yl]-6-iodobenzimidazole Compound [¹²³I] 2: 1-(2-fluoroethyl)-2-[5-{(imidazol-1-yl)methyl}pyridin-3-yl]-6-[¹²³I]iodobenzimidazole (a compound represented by the general formula (2) wherein R₁₂ represents a hydrogen atom; X₁₁ represents a hydrogen atom; X₁₂ represents iodine-123; X₁₄ represents a fluorine atom; and n represents an integer of 2)
Compound 3: 2-[5-{(1H-imidazol-1-yl)methyl}pyridin-3-yl]-6-iodo-1-methyl-1H-benz [d] imidazole Compound [¹²³I] 3: 2-[5-{(1H-imidazol-1-yl)methyl}pyridin-3-yl]-6-[¹²³I]iodo-1-methyl-1H-benz[d]imidazole (a compound represented by the general formula (2) wherein R₁₂ represents a hydrogen atom; X₁₁ represents a hydrogen atom; X₁₂ represents iodine-123; X₁₄ represents a hydrogen atom; and n represents an integer of 1)

Compound 1 was synthesized according to the method for synthesizing Compound 100 in International Publication No. WO 2015/199205.

Compound [¹⁸F] 1 was synthesized according to the method for synthesizing Compound [¹⁸F] 100 in International Publication No. WO 2015/199205, and a compound having 95% or higher radiochemical purity under the TLC conditions disclosed therein was used.

Compound 2 was synthesized according to the method for synthesizing Compound 604 in International Publication No. WO 2015/199205.

Compound [¹²³I] 2 was synthesized according to the method for synthesizing Compound [¹²³I] 604 in International Publication No. WO 2015/199205, and a compound having 95% or higher radiochemical purity under the TLC conditions disclosed therein was used.

Compound 3 was synthesized according to the method for synthesizing Compound 607 in International Publication No. WO 2015/199205.

Compound [¹²³I] 3 was synthesized according to the method for synthesizing Compound [¹²³I] 607 in International Publication No. WO 2015/199205, and a compound having 95% or higher radiochemical purity under the TLC conditions disclosed therein was used.

### (Example 1) CYP11B2 expression evaluation in ischemic heart disease model rat heart

The chest of each Wistar rat (male) was opened under isoflurane anesthesia, and the left coronary artery was ligated for 30 minutes, followed by reperfusion. The chest was closed to prepare an ischemic heart disease model rat. Approximately 1 week after the operation, the rat was sacrificed under anesthesia, and the heart was taken out thereof. Sections of 5 µm in thickness were prepared from the base side of the heart. Immunostaining was carried out using the prepared sections to confirm the expression and distribution of CPY11B2. As an anti-CYP11B2 antibody, one prepared according to the method described in Ogishima T et al., Endocrinology, 1992, vol. 130, pp. 2971-7 was used. As a secondary antibody, HRP Labelled Polymer Anti-Rabbit (manufactured by Dako/Agilent Technologies, Inc.) was used. CYP11B2 expression sites were detected by applying DAB+ (3,3'-diaminobenzidine tetrahydrochloride) substrate kit (manufactured by Dako/Agilent Technologies, Inc.) to the HRP bound to the secondary antibody.

The result is shown in Figure 1. Figure 1 is a view showing a result of immunostaining of CYP11B2. Figure 1(a) is an overall view of the section, Figure 1(b) is a 20-fold enlarged view of a non-ischemic site (ROIa in Figure 1(a)), Figure 1(c) is a 20-fold enlarged view of an ischemia reperfusion site (ROIb in Figure 1(a)), and Figure 1(d) is a 20-fold enlarged view of an ischemia reperfusion site (ROIc in Figure 1(a)). As shown in Figure 1, it was confirmed that CYP11B2 was expressed at the ischemia reperfusion site. It was also confirmed that CYP11B2 was not expressed or was low in expression at the non-ischemic site.

### (Example 2) Ex vivo autoradiography using ischemic heart disease model rat

Each ischemic heart disease model rat was prepared in the same way as in Example 1. Compound [¹⁸F] 1 was administered thereto (approximately 50 MBq/rat) between 1 and 3 weeks after the operation. 20 minutes after the administration, the rat was sacrificed under anesthesia, and the heart was taken out thereof. Frozen sections of 20 µm in thickness were prepared from the base of the heart toward the cardiac apex. The prepared sections were exposed to an imaging plate (BAS-SR2040, manufactured by Fujifilm Corp.) for 2 hours. Autoradiograms were obtained using a fluoro image analyzer (FLA-7000, manufactured by GE Healthcare Corp.).

HE staining, Masson trichrome staining, and the same immunostaining of CYP11B2 as in Example 1 were each carried out using sections adjacent to the section on the base side of the heart.

The results of an individual given Compound [¹⁸F] 1 are shown in Figures 2 and 3. Figure 2 shows the results of autoradiogram of the individual given Compound [¹⁸F] 1. Figure 2(a) shows the respective autoradiograms of sections arranged from the base of the heart toward the cardiac apex. Figure 2(b) is an enlarged view after rotation by 90 degrees of the autoradiogram surrounded with the broken line in Figure 2(a). Figure 3 shows the results of staining a section adjacent to the section shown in Figure 2(b). Figure 3(a) shows the result of HE staining, Figure 3(b) shows the result of Masson trichrome staining, and Figure 3(c) shows the result of immunostaining of CYP11B2.

As illustrated in Figure 2, the local accumulation of Compound [¹⁸F] 1 was confirmed. From the results of staining in Figure 3, inflammatory reaction, fibrosis, and CYP11B2 expression were also confirmed at a position corresponding to the accumulation site of Compound [¹⁸F] 1.

### (Example 3) In vitro autoradiography using ischemic heart disease model rat

Each ischemic heart disease model rat was prepared in the same way as in Example 1. The rat was sacrificed under isoflurane anesthesia between 1 and 3 weeks after the operation. Then, the heart was harvested, and 5 µm sections were prepared and preserved at -80°C until they were used. The sections were brought back to room temperature from -80°C, dried for 30 minutes or longer, then immersed in phosphate-buffered saline for 30 minutes, and subsequently immersed in phosphate-buffered saline containing 1 w/v% bovine serum albumin for 30 minutes for hydrophilization. Each phosphate-buffered saline containing 1 w/v% bovine serum albumin and further containing Compound [¹⁸F] 1 (radioactivity concentration: approximately 40 kBq/mL) or Compound [¹²³I] 2 (radioactivity concentration: approximately 10 kBq/mL) was prepared, and the hydrophilized sections were immersed therein at room temperature for 30 minutes. Then, the sections were washed by immersing for 5 minutes each in phosphate-buffered saline containing 1 w/v% bovine serum albumin, phosphate-buffered saline, and phosphate-buffered saline. The sections thus washed were thoroughly dried and then exposed to light for approximately 3 hours as to Compound [¹⁸F] 1 and for approximately 16 hours as to Compound [¹²³I] 2 on an imaging plate (BAS-SR2040, manufactured by Fujifilm Corp.). Autoradiograms were obtained using a fluoro image analyzer (FLA-7000, manufactured by GE Healthcare Corp.).

Also, autoradiograms were obtained by immersing the sections in a solution containing Compound [¹⁸F] 1 together with 5 µmol/L of Compound 1, or a solution containing Compound [¹²³I] 2 together with 5 µmol/L of Compound 2 in the same way as above.

The same experiment as above was conducted using the heart harvested from a normal rat.

The results of Compound [¹⁸F] 1 are shown in Figure 4. Each of Figures 4(a) and 4(e) shows the section of the normal rat, and each of Figures 4(b) to 4(d) and 4(f) to 4(h) shows the section of the ischemic heart disease model rat. Figures 4(a) to 4(d) resulted from immersing in a solution containing Compound [¹⁸F] 1, and Figures 4(e) to 4(h) resulted from immersing in a solution containing Compound [¹⁸F] 1 and Compound 1 (5 µmol/L). As shown in Figure 4, the accumulation of Compound [¹⁸F] 1 in a lesion region was confirmed. The accumulation was inhibited by the addition of an excessive amount of a non-labeled compound, suggesting that the binding is specific.

The results of Compound [¹²³I] 2 are shown in Figure 5. Each of Figures 5(a) and 5(c) shows the section of the normal rat, and each of Figures 5(b) to 5(d) shows the section of the ischemic heart disease model rat. Figures 5(a) and 5(b) resulted from immersing in a solution containing Compound [¹²³I] 2, and Figures 5(c) and 5(d) resulted from immersing in a solution containing Compound [¹²³I] 2 and Compound 2 (5 µmol/L). As shown in Figure 5, the accumulation of Compound [¹²³I] 2 in a lesion region was confirmed. The accumulation was inhibited by the addition of an excessive amount of a non-labeled compound, suggesting that the binding is specific.

The section of Figure 5(b) was also subjected to staining. The results are shown in Figure 6. Figure 6(a) shows a result of autoradiography. Figure 6(b) shows a result of Masson trichrome staining, and Figure 6(c) shows a result of immunostaining of CYP11B2. As illustrated in Figure 6, the accumulation of Compound [¹²³I] 2 was in good agreement with a fibrosis region. The expression of CYP11B2 at an ischemia reperfusion site was also confirmed.

ROI was established at an ischemia reperfusion site and a non-ischemic site as to the autoradiography of Compound [¹⁸F] 1 and Compound [¹²³I] 2, and the ratio of signal intensity was compared between these sites. The results are shown in Figure 7 (n = 4). Figure 7(a) is one example of an autoradiogram of the section immersed in Compound [¹⁸F] 1. Figure 7(c) is one example of an autoradiogram of the section immersed in Compound [¹²³I] 2. In both Figures 7(a) and 7(c), ROI of the ischemia reperfusion site is surrounded with the solid line, and ROI of the non-ischemic site is surrounded with the broken line. Figure 7(b) is a bar graph that shows a ratio of ischemia reperfusion site signal intensity/non-ischemic site signal intensity of Compound [¹⁸F] 1 in each rat. Figure 7(d) is a bar graph that shows a ratio of ischemia reperfusion site signal intensity/non-ischemic site signal intensity of Compound [¹²³I] 2 in each rat. As shown in Figure 7, the increased accumulation of Compound [¹⁸F] 1 and Compound [¹²³I] 2 at the ischemia reperfusion site compared with the non-ischemic site was observed. Higher accumulation of Compound [¹²³I] 2 than that of Compound [¹⁸F] 1 was also observed.

### (Example 4) Study on influence of elapsed time after ischemic heart disease model rat preparation on compound accumulation

Each ischemic heart disease model rat was prepared in the same way as in Example 1. The rat was sacrificed under isoflurane anesthesia 1 day (3 rats), 3 days (4 rats) or 1 week (4 rats) after the operation. Then, the heart was harvested, and 5 µm-thick sections were prepared and preserved at -80°C until they were used. The sections were brought back to room temperature from -80°C, dried for 30 minutes or longer, then immersed in phosphate-buffered saline for 30 minutes, and subsequently immersed in phosphate-buffered saline containing 1 w/v% bovine serum albumin for 30 minutes for hydrophilization. Phosphate-buffered saline containing 1 w/v% bovine serum albumin and further containing Compound [¹²³I] 2 (radioactivity concentration: approximately 10 kBq/mL) was prepared, and the hydrophilized sections were immersed therein at room temperature for 30 minutes. Then, the sections were washed by immersing for 5 minutes each in phosphate-buffered saline containing 1 w/v% bovine serum albumin, phosphate-buffered saline, and phosphate-buffered saline. The sections thus washed were thoroughly dried and then exposed to light for approximately 16 hours on an imaging plate (BAS-SR2040, manufactured by Fujifilm Corp.). Autoradiograms were obtained using a fluoro image analyzer (FLA-7000, manufactured by GE Healthcare Corp.).

The same experiment as above was conducted using the hearts harvested from normal rats (two rats).

The results are shown in Figure 8. Figure 8(a) is a bar graph that shows a ratio of ischemia reperfusion site signal intensity/non-ischemic site signal intensity of Compound [¹²³I] 2 in autoradiography using the hearts of the normal rat and the rats 1 day, 3 days and 1 week after ischemic heart disease model rat preparation. Figure 8(b) shows the results of Masson trichrome staining using sections adjacent to the sections used in Figure 8(a). As shown in Figure 8(a), the accumulation of Compound [¹²³I] 2 was increased with elapsed time after the operation. As shown in Figure 8(b), the acceleration of fibrosis was also confirmed with elapsed time after the operation.

### (Example 5) SPECT imaging experiment using ischemic heart disease model rat

Each ischemic heart disease model rat was prepared in the same way as in Example 1. Compound [¹²³I] 2 was administered thereto (approximately 100 MBq/rat) in one week after the operation. Static imaging for approximately 8 minutes was started at 150 minutes after the administration using a SPECT apparatus for animals (FX3000, manufactured by TriFoil Imaging). Data collection was carried out in an energy window of 143 to 175 keV, and the collected data was reconstituted by OSEM (Ordered Subset Expectation Maximization) to obtain images. Computed tomography imaging was carried out in order to identify the position of the heart. The same experiment as above was conducted using a normal rat.

The results are shown in Figure 9. Figures 9(a) to 9(c) show the results of SPECT imaging based on Compound [¹²³I] 2 using the normal rat. In Figures 9(a) to 9(c), the arrow H depicts the heart, and the arrow L depicts the liver. Figures 9(d) to 9(f) show the results of SPECT imaging based on Compound [¹²³I] 2 using the ischemic heart disease model rat. In Figures 9(d) to 9(f), the arrow i depicts an ischemia reperfusion site. Each of Figures 9(a) and 9(d) is a short axis image, each of Figures 9(b) and 9(e) is a horizontal long axis image, and each of Figures 9(c) and 9(f) is a vertical long axis image. All the SPECT images of Figure 9 were displayed in a manner superimposed with a computed tomography image. As shown in Figure 9, the accumulation of Compound [¹²³I] 2, which was not observed in the heart of the normal rat, was confirmed in the heart of the ischemic heart disease model rat.

### (Example 6) CYP11B2 expression evaluation in myocarditis model rat heart

Porcine heart cardiac myosin (Sigma-Aldrich Co. LLC) was diluted to 5 mg/mL using a phosphate buffer solution (solution A). 100 mg of *Mycobacterium tuberculosis* H37Ra (Difco Laboratories Ltd.) was added to 10 mL of Freund's Adjuvant, Complete (Sigma-Aldrich Co. LLC) and mixed therewith (solution B). Solution A and solution B were mixed at a ratio of 1:1 until becoming uniform (solution C). Solution C was administered at 50 µL each to the right and left hind footpads of each Lewis rat (male, 7 weeks old, Charles River Laboratories Japan, Inc.) under isoflurane anesthesia. After raising up to 21 days after the immunization, the rat was sacrificed under anesthesia, and the heart was taken out thereof. Sections of 5 µm in thickness were prepared. Immunostaining was carried out using the prepared sections to confirm the expression and distribution of CPY11B2. As an anti-CYP11B2 antibody, one prepared according to the method described in Ogishima T et al., Endocrinology, 1992, vol. 130, pp. 2971-7 was used. As a secondary antibody, HRP Labelled Polymer Anti-Rabbit (manufactured by Dako/Agilent Technologies, Inc.) was used. CYP11B2 expression sites were detected by applying DAB+ (3,3'-diaminobenzidine tetrahydrochloride) substrate kit (manufactured by Dako/Agilent Technologies, Inc.) to the HRP bound to the secondary antibody. The same experiment as above was conducted using the heart harvested from a conventionally raised Lewis rat (normal rat).

The results are shown in Figure 10. Figure 10 is a view showing the results of immunostaining of CYP11B2. Figure 10(a) is an overall view of the heart tissue section of the myocarditis model, and Figure 10(b) is a 40-fold enlarged view of the lesion site in Figure 10(a). Figure 10(c) is an overall view of the heart tissue section of the normal rat, and Figure 10(d) is a 40-fold enlarged view of the normal site in Figure 10(c). As shown in Figure 10, it was confirmed that CYP11B2 was expressed in the heart of the myocarditis model rat. It was also confirmed that CYP11B2 was not expressed or was low in expression in the heart of the normal rat.

### (Example 7) In vitro autoradiography using myocarditis model rat

Each myocarditis model rat was prepared in the same way as in Example 6. The heart was harvested, and 5 µm-thick sections were prepared and preserved at -80°C until they were used. The sections were brought back to room temperature from -80°C, dried for 30 minutes or longer, then immersed in phosphate-buffered saline for 30 minutes, and subsequently immersed in phosphate-buffered saline containing 1 w/v% bovine serum albumin for 30 minutes for hydrophilization. Phosphate-buffered saline containing 1 w/v% bovine serum albumin and further containing Compound [¹²³I] 3 (radioactivity concentration: approximately 10 kBq/mL) was prepared, and the hydrophilized sections were immersed therein at room temperature for 30 minutes. Then, the sections were washed by immersing for 5 minutes each in phosphate-buffered saline containing 1 w/v% bovine serum albumin, phosphate-buffered saline, and phosphate-buffered saline. The sections thus washed were thoroughly dried and then exposed to light for approximately 16 hours as to Compound [¹²³I] 3 on an imaging plate (BAS-SR2040, manufactured by Fujifilm Corp.). Autoradiograms were obtained using a fluoro image analyzer (FLA-7000, manufactured by GE Healthcare Corp.). The same experiment as above was conducted using the heart harvested from a normal rat. Masson trichrome staining and the same immunostaining of CYP11B2 as in Example 6 were each carried out using sections adjacent to the sections used in autoradiography.

The results of *in vitro* autoradiography of Compound [¹²³I] 3 are shown in Figure 11. Figure 11(a) shows the result of autoradiography of the heart tissue section of the normal rat, and Figure 11(b) shows the result of autoradiography of the heart tissue section of the myocarditis model rat. Figure 11(c) shows the result of Masson trichrome staining of the heart tissue section of the normal rat, and Figure 11(d) shows the result of Masson trichrome staining of the heart tissue section of the myocarditis model rat. Figure 11(e) shows the result of immunostaining of CYP11B2 of the heart tissue section of the normal rat, and Figure 11(f) shows the result of immunostaining of CYP11B2 of the heart tissue section of the myocarditis model rat. As illustrated in Figure 11, the increased accumulation of Compound [¹²³I] 3 in the heart tissue section of the myocarditis model rat compared with the heart tissue section of the normal rat was confirmed. The accumulation of Compound [¹²³I] 3 was in good agreement with a fibrosis region, and the expression of CYP11B2 in the fibrosis region was also confirmed.

### (Example 8) CYP11B2 expression evaluation in hypertensive heart disease model rat heart

Each DIS/Eis (Dahl-Iwai S) rat (male, Japan SLC, Inc.) was fed with 8% NaCl diet (Oriental Yeast Co., Ltd.) from the age of 5 weeks, raised up to the age of 11 weeks, and then sacrificed under anesthesia, and the heart was taken out thereof. Sections of 5 µm in thickness were prepared. Immunostaining was carried out using the prepared sections to confirm the expression and distribution of CPY11B2. As an anti-CYP11B2 antibody, one prepared according to the method described in Ogishima T et al., Endocrinology, 1992, vol. 130, pp. 2971-7 was used. As a secondary antibody, HRP Labelled Polymer Anti-Rabbit (manufactured by Dako/Agilent Technologies, Inc.) was used. CYP11B2 expression sites were detected by applying DAB+ (3,3'-diaminobenzidine tetrahydrochloride) substrate kit (manufactured by Dako/Agilent Technologies, Inc.) to the HRP bound to the secondary antibody. The same experiment as above was conducted using the heart harvested from a DIS/Eis rat raised with a NaCl-free diet (normal rat).

The results are shown in Figure 12. Figure 12 is a view showing the results of immunostaining of CYP11B2. Figure 12(a) is an overall view of the heart tissue section of the hypertensive heart disease model rat, and Figure 12(b) is a 40-fold enlarged view of the lesion site in Figure 12(a). Figure 12(c) is an overall view of the heart tissue section of the normal rat, and Figure 12(d) is a 40-fold enlarged view of the normal site in Figure 12(c). As shown in Figure 12, it was confirmed that CYP11B2 was expressed in the heart of the hypertensive heart disease model rat. It was also confirmed that CYP11B2 was not expressed or was low in expression in the normal rat.

### (Example 9) In vitro autoradiography using hypertensive heart disease model rat

Each hypertensive heart disease model rat was prepared in the same way as in Example 8. The heart was harvested, and 5 µm-thick sections were prepared and preserved at -80°C until they were used. The sections were brought back to room temperature from -80°C, dried for 30 minutes or longer, then immersed in phosphate-buffered saline for 30 minutes, and subsequently immersed in phosphate-buffered saline containing 1 w/v% bovine serum albumin for 30 minutes for hydrophilization. Phosphate-buffered saline containing 1 w/v% bovine serum albumin and further containing Compound [¹²³I] 3 (radioactivity concentration: approximately 10 kBq/mL) was prepared, and the hydrophilized sections were immersed therein at room temperature for 30 minutes. Then, the sections were washed by immersing for 5 minutes each in phosphate-buffered saline containing 1 w/v% bovine serum albumin, phosphate-buffered saline, and phosphate-buffered saline. The sections thus washed were thoroughly dried and then exposed to light for approximately 16 hours as to Compound [¹²³I] 3 on an imaging plate (BAS-SR2040, manufactured by Fujifilm Corp.). Autoradiograms were obtained using a fluoro image analyzer (FLA-7000, manufactured by GE Healthcare Corp.). The same experiment as above was conducted using the heart harvested from a normal rat. Masson trichrome staining and the same immunostaining of CYP11B2 as in Example 8 were each carried out using sections adjacent to the sections used in autoradiography.

The results of *in vitro* autoradiography of Compound [¹²³I] 3 are shown in Figure 13. Figure 13(a) shows the result of autoradiography of the heart tissue section of the normal rat, and Figure 13(b) shows the result of autoradiography of the heart tissue section of the hypertensive heart disease model rat. Figure 13(c) shows the result of Masson trichrome staining of the heart tissue section of the normal rat, and Figure 13(d) shows the result of Masson trichrome staining of the heart tissue section of the hypertensive heart disease model rat. Figure 13(e) shows the result of immunostaining of CYP11B2 of the heart tissue section of the normal rat, and Figure 13(f) shows the result of immunostaining of CYP11B2 of the heart tissue section of the hypertensive heart disease model rat. As illustrated in Figure 13, the increased accumulation of Compound [¹²³I] 3 in the heart tissue section of the hypertensive heart disease model rat compared with the heart tissue section of the normal rat was confirmed. The accumulation of Compound [¹²³I] 3 was in good agreement with a fibrosis region, and the expression of CYP11B2 in the fibrosis region was also confirmed.

### (Example 10) In vitro autoradiography using ischemic heart disease model rat

Each ischemic heart disease model rat was prepared in the same way as in Example 1. The rat was sacrificed under isoflurane anesthesia in one week after the operation. Then, the heart was harvested, and 5 µm-thick sections were prepared and preserved at -80°C until they were used. The sections were brought back to room temperature from -80°C, dried for 30 minutes or longer, then immersed in phosphate-buffered saline for 30 minutes, and subsequently immersed in phosphate-buffered saline containing 1 w/v% bovine serum albumin for 30 minutes for hydrophilization. Phosphate-buffered saline containing 1 w/v% bovine serum albumin and further containing Compound [¹²³I] 3 (radioactivity concentration: approximately 10 kBq/mL) was prepared, and the hydrophilized sections were immersed therein at room temperature for 30 minutes. Then, the sections were washed by immersing for 5 minutes each in phosphate-buffered saline containing 1 w/v% bovine serum albumin, phosphate-buffered saline, and phosphate-buffered saline. The sections thus washed were thoroughly dried and then exposed to light for approximately 16 hours on an imaging plate (BAS-SR2040, manufactured by Fujifilm Corp.). Autoradiograms were obtained using a fluoro image analyzer (FLA-7000, manufactured by GE Healthcare Corp.). The same experiment as above was conducted using the heart harvested from a normal rat.

The results are shown in Figure 14. Figure 14(a) resulted from immersing of the section of the normal rat in a solution containing Compound [¹²³I] 3, and Figure 14(b) resulted from immersing of the section of the ischemic heart disease model rat in a solution containing Compound [¹²³I] 3. As shown in Figure 14, the increased accumulation of Compound [¹²³I] 3 in a lesion region compared with the normal rat was confirmed.

The results described above suggested that the radioactively labeled compound capable of binding to an aldosterone synthase achieves nuclear medicine diagnosis of the myocardial remodeling process such as the progression of fibrosis in heart disease patients.

This application claims the priority based on Japanese Patent Application No. 2016-115806 filed on June 10, 2016, the disclosure of which is incorporated herein by reference in its entirety.

## Claims

1. A non-invasive diagnostic imaging agent for a heart disease, comprising a radioactively labeled compound capable of binding to an aldosterone synthase or a salt thereof as an active ingredient.

2. The non-invasive diagnostic imaging agent according to claim 1, wherein the radioactively labeled compound is labeled with carbon-11, fluorine-18, chlorine-34m, bromine-76, iodine-123 or iodine-124.

3. The non-invasive diagnostic imaging agent according to claim 1, wherein the radioactively labeled compound is represented by the following general formula (1) : 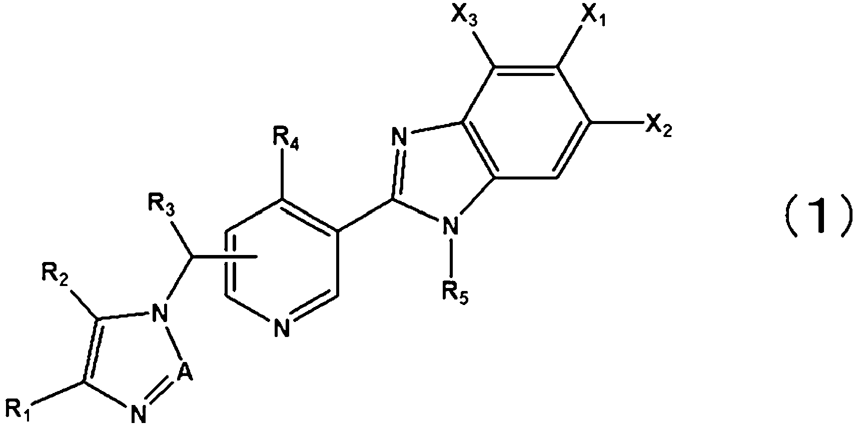 wherein R₁ represents a hydrogen atom or CO₂Rₐ; R₂ represents a hydrogen atom, a halogen atom or CO₂Rₐ; R₃ represents a hydrogen atom or a hydroxyalkyl group having 1 to 10 carbon atoms; R₄ represents a hydrogen atom, a hydroxy group or an alkoxy group having 1 to 10 carbon atoms; R₅ represents a chain alkyl group having 1 to 5 carbon atoms in which a hydrogen atom is optionally replaced with a halogen atom, a cyclic alkyl group having 3 to 5 carbon atoms in which a hydrogen atom is optionally replaced with a halogen atom, a hydroxyalkyl group having 1 to 5 carbon atoms, or an o-, p- or m-halobenzyl group; A represents CH or a nitrogen atom; X₁ and X₃ each independently represent a hydrogen atom or a halogen atom, and X₂ represents a hydrogen atom, a halogen atom or a nitrile group, provided that at least one of X₁, X₂ and X₃ is a halogen atom; each Rₐ independently represents an alkyl group having 1 to 10 carbon atoms; and any one of R₂, R₅, and X₂ contains a radioactive halogen atom.

4. The non-invasive diagnostic imaging agent according to claim 3, wherein the radioactively labeled compound is represented by the following general formula (2) : 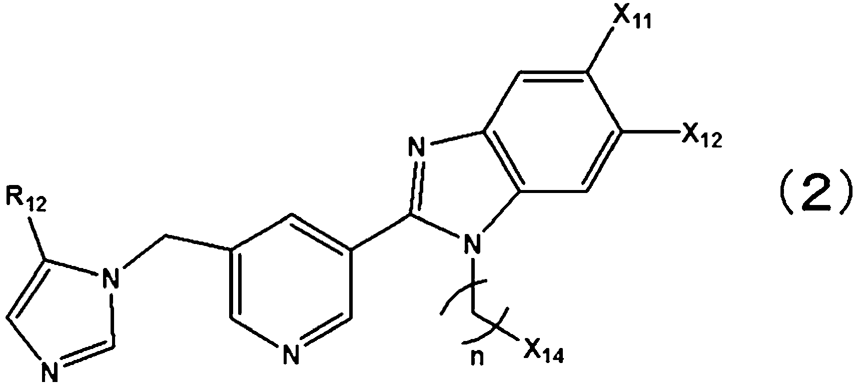 wherein R₁₂ represents a hydrogen atom, a halogen atom or CO₂Rₐ; X₁₁ represents a hydrogen atom or a halogen atom; X₁₂ represents a halogen atom; X₁₄ represents a hydrogen atom, a halogen atom or a hydroxy group; n represents an integer of 1 to 5; Rₐ represents an alkyl group having 1 to 10 carbon atoms; and R₁₂, X₁₂, or X₁₄ is a radioactive halogen atom.

5. The non-invasive diagnostic imaging agent according to claim 4, wherein in the general formula (2), R₁₂ is a hydrogen atom, and X₁₂ or X₁₄ is a radioactive halogen atom.

6. The non-invasive diagnostic imaging agent according to claim 4 or 5, wherein in the general formula (2), n represents an integer of 1 to 3.

7. The non-invasive diagnostic imaging agent according to any one of claims 3 to 6, wherein the radioactive halogen atom is fluorine-18, chlorine-34m, bromine-76, iodine-123 or iodine-124.

8. The non-invasive diagnostic imaging agent according to any one of claims 1 to 7 for use in positron emission tomography or single-photon emission computed tomography.

9. The non-invasive diagnostic imaging agent according to any one of claims 1 to 8, wherein the heart disease is an ischemic heart disease.

10. The non-invasive diagnostic imaging agent according to claim 9, wherein the ischemic heart disease is a coronary heart disease angina pectoris, myocardial infarction, acute coronary syndrome or ischemic heart failure.

11. The non-invasive diagnostic imaging agent according to any one of claims 1 to 8, wherein the heart disease is a non-ischemic heart disease.

12. The non-invasive diagnostic imaging agent according to claim 11, wherein the non-ischemic heart disease is myocarditis, hypertensive heart disease, dilated cardiomyopathy, hypertrophic cardiomyopathy or non-ischemic heart failure.
